(19) Europäisches Patentamt / European Patent Office / Office européen des brevets

(11) **EP 3 489 347 A1**

(12) **EUROPEAN PATENT APPLICATION**
published in accordance with Art. 153(4) EPC

(43) Date of publication:
**29.05.2019 Bulletin 2019/22**

(21) Application number: **17834367.9**

(22) Date of filing: **25.07.2017**

(51) Int Cl.:
*C12M 3/00* (2006.01)     *C12N 5/071* (2010.01)

(86) International application number:
**PCT/JP2017/026945**

(87) International publication number:
**WO 2018/021365 (01.02.2018 Gazette 2018/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **25.07.2016   JP 2016145833**

(71) Applicant: UBE Industries, Ltd.
**Ube-shi, Yamaguchi 755-8633 (JP)**

(72) Inventors:
• **HAGIHARA, Masahiko**
  **Ube-shi**
  **Yamaguchi 755-8633 (JP)**
• **FUSE, Shinsaku**
  **Ube-shi**
  **Yamaguchi 755-8633 (JP)**
• **SHIMIZU, Motohisa**
  **Ube-shi**
  **Yamaguchi 755-8633 (JP)**
• **WADA, Yukinori**
  **Ube-shi**
  **Yamaguchi 755-8633 (JP)**

(74) Representative: **Plougmann Vingtoft a/s**
**Strandvejen 70**
**2900 Hellerup (DK)**

(54) **CELL CULTIVATION DEVICE AND CELL CULTIVATION METHOD USING SAME**

(57)     The present invention provides a cell cultivation device comprising: a polymer porous film; a cell cultivation part that accommodates the polymer porous film; a culture medium supply means that is positioned at an upper section of the cell cultivation part; and a culture medium recovery means that is positioned at a lower section of the cell cultivation part, wherein the polymer porous film is a polymer porous film with a three-layer structure, having a surface layer A and a surface layer B that have a plurality of holes, and a macrovoid layer that is sandwiched between the surface layer A and the surface layer B, the average hole diameter of the holes present in the surface layer A is smaller than the average hole diameter of the holes present in the surface layer B, the macrovoid layer has dividing walls that are connected to the surface layers A and B, and a plurality of macrovoids that are surrounded by the dividing walls and the surface layers A and B, the holes in the surface layers A and B are in communication with the macrovoids, and the cell cultivation part comprises a side part and a bottom part that has one or more culture medium discharge ports.

FIG. 2

**Description**

FIELD

The present invention relates to a cell culture apparatus provided with a porous polymer film. In addition, it relates to a cell culture method using a cell culture apparatus provided with a porous polymer film.

BACKGROUND

In recent years, proteins such as enzymes, hormones, antibodies, cytokines, viruses (viral proteins) used for treatment and vaccine are industrially produced using cultured cells. However, such a protein production technology is expensive, raising medical cost. Accordingly, there have been demands for innovating technologies for culturing cells at high density and for increasing protein production, aiming at great reduction of cost.

As cells for protein production, anchorage-dependent adherent cells which adhere to a culture substrate may be sometimes used. Since such cells grow anchorage-dependently, they need to be cultured while being adhered onto the surface of a dish, plate or chamber. Conventionally, in order to culture such adherent cells in a large amount, it was preferable to increase the surface area to be adhered. However, increasing the culturing area inevitably requires to increase the space, which is responsible for increase in cost.

As a method to culture a large amount of adherent cells while decreasing the culture space, a culture method using a carrier having micropores, especially a microcarrier, has been developed (for example, PTL 1). In a cell culturing system using microcarriers, it is preferable to carry out sufficient stirring and diffusion so that the microcarriers do not aggregate together. Since this requires a volume allowing adequate agitation and diffusion of the medium in which the microcarriers are dispersed, there is an upper limit to the density at which the cells can be cultured. In order to separate the microcarrier from the medium, separation is preferably performed using a filter which can separate fine particles, possibly resulting in increased cost. Considering the foregoing, there is a demand for innovative methodology for cell culture which cultures cells at high density.

<Porous Polyimide Film>

Porous polyimide films have been utilized in the prior art for filters and low permittivity films, and especially for battery-related purposes, such as fuel cell electrolyte membrane and the like. PTLs 2 to 4 describe porous polyimide films with numerous macrovoids, having excellent permeability to objects such as gases, high porosity, excellent smoothness on both surfaces, relatively high strength and, despite high porosity, excellent resistance against compression stress in the film thickness direction. All of these are porous polyimide films formed via amic acid.

The cell culture method which includes applying cells to a porous polyimide film and culturing them is reported (PTL 5).

[CITATION LIST]

[PATENT LITERATURE]

[PTL 1] WO2003/054174
[PTL 2] WO2010/038873
[PTL 3] Japanese Unexamined Patent Publication (Kokai) No. 2011-219585
[PTL 4] Japanese Unexamined Patent Publication (Kokai) No. 2011-219586
[PTL 5] WO2015/012415

SUMMARY

[TECHNICAL PROBLEM]

The present invention aims at providing a cell culture apparatus provided with a porous polymer film. In addition, the present invention also aims at providing a cell culture method using a cell culture apparatus provided with a porous polymer film.

[SOLUTION TO PROBLEM]

The present inventors have found that a porous polymer film having a predetermined structure provides not only an optimum space capable of culturing a large amount of cells but also a wet environment resistant to drying. Accordingly, we have completed an apparatus for culturing cells while exposing them to a gas phase, and a culture method using such an apparatus. In other words, the present invention preferably includes, but is not limited to, the following modes.

[1] A cell culture apparatus comprising:

a porous polymer film;
a cell culture section containing the porous polymer film;
a medium supplying means disposed at a upper portion of the cell culture section; and
a medium collecting means disposed at a lower portion of the cell culture section, wherein the porous polymer films are a three-layer structure porous polymer film having a surface layer A and a surface layer B, the surface layers having a plurality of pores, and a macrovoid layer sandwiched between the surface layers A and B;

wherein an average pore diameter of the pores present in the surface layer A is smaller than an average pore diameter of the pores present in the surface layer B;
wherein the macrovoid layer has a partition wall bonded to the surface layers A and B, and a plurality of macrovoids surrounded by such a partition wall and the surface layers A and B;
wherein the pores in the surface layers A and B communicate with the macrovoid; and
wherein the cell culture section comprises a bottom portion having one or more medium outlets, and a side portion disposed approximately perpendicular to the bottom portion.
[2] The cell culture apparatus according to [1], wherein two or more cell culture sections are stacked.
[3] The cell culture apparatus according to [1] or [2], wherein the side portion has one or more additional medium inlets.
[4] The cell culture apparatus according to any one of [1] to [3], the apparatus characterized by further comprising:

a medium discharging line communicating with the medium collecting means at one end;
a medium storage tank communicating with the other end of the medium discharging line; and
a medium supplying line communicating with the medium storage tank at one end;

wherein the other end of the medium supplying line communicates with the medium supplying means, and the medium circulates.
[5] The cell culture apparatus according to [4], the apparatus further comprising a pump for pumping up the medium in the medium storage tank into the medium supplying means.
[6] The cell culture apparatus according to any one of [1] to [5], wherein the medium collecting means is funnel-shaped.
[7] The cell culture apparatus according to any one of [1] to [6], wherein the medium supplying means has a medium storage section, and two or more medium dropping nozzles provided at the bottom portion of the medium storage section.
[8] The cell culture apparatus according to any one of [1] to [6], wherein the medium supplying means is a medium droplet supplying means.
[9] The cell culture apparatus according to any one of [1] to [8], the apparatus further comprising:

an outer cylinder for housing the cell culture section;
wherein the medium supplying means is disposed in the outer cylinder and above the cell culture section;
wherein the culture medium collecting means is disposed in the outer cylinder and under the cell culture section.

[10] The cell culture apparatus according to any one of [1] to [9], wherein the porous polymer film is contained in the cell culture section, with the porous polymer film:

i) being folded up;
ii) being wound into a roll-like shape;
iii) sheets or pieces thereof being concatenated with a thread-like structure;
iv) being tied together into a rope-like shape; and/or
v) two or more thereof being stacked.

[11] The cell culture apparatus according to any one of [1] to [9], wherein the porous polymer film is a modularized porous polymer film having a casing;

wherein the modularized porous polymer film is contained within the casing with:

(i) the two or more independent porous polymer films being aggregated;
(ii) the porous polymer film being folded up;
(iii) the porous polymer film being wound into a roll-like shape; and/or
(iv) the porous polymer film being tied together into a rope-like shape;

wherein the modularized porous polymer film is contained in the cell culture section.

[12] The cell culture apparatus according to any one of [1] to [11], wherein the porous polymer film has a plurality of pores having an average pore diameter of 0.01 to 100 μm.

[13] The cell culture apparatus according to any one of [1] to [12], wherein an average pore diameter of the surface layer A is 0.01 to 50 μm.

[14] The cell culture apparatus according to any one of [1] to [13], wherein an average pore diameter of the surface layer B is 20 to 100 μm.

[15] The cell culture apparatus according to any one of [1] to [14], wherein a total film thickness of the porous polymer film is 5 to 500 μm.

[16] The cell culture apparatus according to any one of [1] to [15], wherein the porous polymer film is a porous polyimide film.

[17] The cell culture apparatus according to [16], wherein the porous polyimide film is a porous polyimide film comprising a polyimide derived from tetracarboxylic dianhydride and diamine.

[18] The cell culture apparatus according to [16] or [17], wherein the porous polyimide film is a colored porous polyimide film that is obtained by molding a polyamic acid solution composition comprising a polyamic acid solution derived from tetracarboxylic dianhydride and diamine, and a coloring precursor, and subsequently heat-treating the resultant composition at 250°C or higher.

[19] The cell culture apparatus according to any one of [1] to [15], wherein the porous polymer film is a porous polyethersulfone film.

[20] A method for culturing a cell which uses the cell culture apparatus according to any one of [1] to [19].

[21] A cell culture apparatus comprising:

a porous polymer film;
a cell culture section containing the porous polymer film;
a medium supplying means disposed at a upper portion of the cell culture section; and
a medium collecting means disposed at a lower portion of the cell culture section,

wherein the porous polymer films are a three-layer structure porous polymer film having a surface layer A and a surface layer B, the surface layers having a plurality of pores, and a macrovoid layer sandwiched between the surface layers A and B;

wherein an average pore diameter of the pores present in the surface layer A is smaller than an average pore diameter of the pores present in the surface layer B;

wherein the macrovoid layer has a partition wall bonded to the surface layers A and B, and a plurality of macrovoids surrounded by such a partition wall and the surface layers A and B;

wherein the pores in the surface layers A and B communicate with the macrovoid; and

wherein the medium collecting means is a part of an outer cylinder for housing the cell culture section.

[22] The cell culture apparatus according to [21], wherein the medium supplying means is a medium droplet supplying means.

[23] The cell culture apparatus according to [21] or [22], wherein the porous polymer film is a modularized porous polymer film having a casing;

wherein the modularized porous polymer film is contained within the casing with:

(i) the two or more independent porous polymer films being aggregated;
(ii) the porous polymer film being folded up;
(iii) the porous polymer film being wound into a roll-like shape; and/or
(iv) the porous polymer film being tied together into a rope-like shape;

wherein the modularized porous polymer film is contained in the cell culture section.

[ADVANTAGEOUS EFFECTS OF INVENTION]

The present invention can reduce space for culture by using a porous polymer film as a cell culture carrier. In addition, employing a cell culture apparatus of the present invention enables simple and efficient cell culture even under conditions including less amount of a medium. In addition, since a porous polymer film has a low hydrophilic porous property and liquid can be stably held in the porous polymer film, a wet environment is provided that is resistant to drying. It is therefore possible to achieve survival and proliferation of cells even in very small amounts of medium, even when compared with conventional cell culturing apparatuses. Furthermore, it is possible to carry out culturing even if all or some of the porous polymer film has been exposed to air, oxygen can be efficiently supplied to the cells, and is suitable for mass culturing of cells.

An amount of a medium to be used can be greatly reduced by employing a cell culture apparatus of the present invention. In addition, a porous polymer film used for a cell culture apparatus of the present invention makes it possible to culture while exposing cells to a gas phase. Therefore, oxygen supply to cells supported on the porous polymer film can be sufficiently attained by diffusion. Accordingly, a cell culture apparatus of the present invention requires no separate means for oxygen supply. Moreover, since a cell culture apparatus of the present invention has a supplying means for supplying a dropletized medium from an arbitrary direction to the porous polymer film, it is possible to supply a homogeneous medium to cells existing at arbitrary site of the porous polymer film or cell culture module.

BRIEF DESCRIPTION OF DRAWINGS

FIG. 1 represents a diagram illustrating a cell culture section in an embodiment. The upper panel represents a planar view, and the lower panel represents a side view.

FIG. 2 is a cross sectional view illustrating a configurational example of a cell culture apparatus according to an embodiment of the invention.

FIG. 3 represents a diagram illustrating a cell culture supplying means in an embodiment. (A) is a planar view, (B) is a cross sectional view, and (C) is a perspective view.

FIG. 4 is a perspective view illustrating a configurational example of a cell culture apparatus according to an embodiment.

FIG. 5 is a cross sectional view illustrating a configurational example of a cell culture apparatus according to an embodiment.

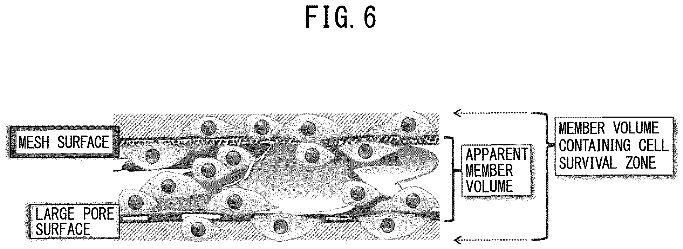

FIG. 6 is a model diagram of cell culturing using a porous polymer film.

FIG. 7 is a diagram illustrating a configurational example of a cell culture apparatus according to an embodiment of the invention.

FIG. 8 represents a conceptual diagram illustrating embodiments of medium dropped from a medium droplet supplying means in a cell culture supplying means according to an embodiment of the invention. (A) represents a drop-type, (B) represents a mesh-type, and (C) represents a shower-type. (D) represents a diagram illustrating a configuration of a lid body of an outer cylinder used for supplying drop-type and mesh-type droplets. A stainless steel mesh wound into a mesh bundle is inserted in a medium supplying port of the lid body of the outer cylinder.

FIG. 9 is a diagram illustrating a configurational example of a cell culture apparatus according to an embodiment of the invention. (A) is a schematic diagram, and (B) is a photograph representing an appearance of the actual cell culture apparatus.

FIG. 10 is a diagram illustrating a modularized porous polymer film applied to a cell culture apparatus according to an embodiment of the invention.

FIG. 11 is a graph illustrating an amount of fibronectin produced from a human skin fibroblast when a cell culture apparatus according to an embodiment of the present invention which is illustrated in Example 6 is used.

DESCRIPTION OF EMBODIMENTS

The embodiments of the present invention will be hereinafter described with reference to drawings as needed. The configuration of the embodiments is illustrative, and the configuration of the present invention is not limited to the specific configuration of the embodiment.

1. Porous polymer film

The average pore diameter of the pore present on a surface layer A (hereinafter referred to as "surface A" or "mesh surface") in the porous polymer film used for the present invention is not particularly limited, but is, for example,

0.01 μm or more and less than 200 μm, 0.01 to 150 μm, 0.01 to 100 μm, 0.01 to 50 μm, 0.01 to 40 μm, 0.01 to 30 μm, 0.01 to 20 μm, or 0.01 to 15 μm, preferably 0.01 to 15 μm.

The average pore diameter of the pore present on a surface layer B (hereinafter referred to as "surface B" or "large pore surface") in the porous polymer film used for the present invention is not particularly limited so long as it is larger than the average pore diameter of the pore present on the surface A, but is, for example, greater than 5 μm and 200 μm or less, 20 μm to 100 μm, 30 μm to 100 μm, 40 μm to 100 μm, 50 μm to 100 μm, or 60 μm to 100 μm, preferably 20 μm to 100 μm.

The average pore diameter on the surface of the porous polymer film is determined by measuring pore area for 200 or more open pore portions, and calculated an average diameter according to the following Equation (1) from the average pore area assuming the pore shape as a perfect circle.

[Math 1]

$$\text{Average Pore Diameter} = 2 \times \sqrt{(Sa/\pi)} \qquad (1)$$

(wherein Sa represents the average value for the pore areas).

The thicknesses of the surface layers A and B is not particularly limited, but is, for example, 0.01 to 50 μm, preferably 0.01 to 20 μm.

The average pore diameter of macrovoids in the planar direction of the film in the macrovoid layer in the porous polymer film is not particularly limited but is, for example, 10 to 500 μm, preferably 10 to 100 μm, and more preferably 10 to 80 μm. The thicknesses of the partition wall in the macrovoid layer is not particularly limited, but is, for example, 0.01 to 50 μm, preferably 0.01 to 20 μm. In an embodiment, at least one partition wall in the macrovoid layer has one or two or more pores connecting the neighboring macrovoids and having the average pore diameter of 0.01 to 100 μm, preferably 0.01 to 50 μm. In another embodiment, the partition wall in the macrovoid layer has no pore.

The total film thickness of the porous polymer film used for the invention is not particularly limited, but may be 5 μm or more, 10 μm or more, 20 μm or more or 25 μm or more, and 500 μm or less, 300 μm or less, 100 μm or less, 75 μm or less, or 50 μm or less. It is preferably 5 to 500 μm, and more preferably 25 to 75 μm.

The film thickness of the porous polymer film used for the invention can be measured using a contact thickness gauge.

The porosity of the porous polymer film used in the present invention is not particularly limited but is, for example, 40% or more and less than 95%.

The porosity of the porous polymer film used for the invention can be determined by measuring the film thickness and mass of the porous film cut out to a prescribed size, and performing calculation from the basis weight according to the following Equation (2).

[Math 2]

$$\text{Porosity} (\%) = (1 - w/(S \times d \times D)) \times 100 \qquad (2)$$

(wherein S represents the area of the porous film, d represents the total film thickness, w represents the measured mass, and D represents the polymer density. The density is defined as 1.34 g/cm$^3$ when the polymer is a polyimide.)

The porous polymer film used for the present invention is preferably a porous polymer film which includes a three-layer structure porous polymer film having a surface layer A and a surface layer B, the surface layers having a plurality of pores, and a macrovoid layer sandwiched between the surface layers A and B; wherein the average pore diameter of the pore present on the surface layer A is 0.01 μm to 15 μm, and the average pore diameter of the pore present on the surface layer B is 20 μm to 100 μm; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 μm; wherein the pores on the surface layers A and B communicate with the macrovoid, the total film thickness is 5 to 500 μm, and the porosity is 40% or more and less than 95%. In an embodiment, at least one partition wall in the macrovoid layer has one or two or more pores communicating the neighboring macrovoids with each other and having the average pore diameter of 0.01 to 100 μm, preferably 0.01 to 50 μm. In another embodiment, the partition wall has does not have such pores.

The porous polymer film used in the invention is preferably sterilized. The sterilization treatment is not particularly limited, but any sterilization treatment such as dry heat sterilization, steam sterilization, sterilization with a disinfectant such as ethanol, electromagnetic wave sterilization such as ultraviolet rays or gamma rays, and the like can be mentioned.

The porous polymer film used for the present invention has the structural features described above and is not particularly limited but includes, preferably a porous polyimide film or porous polyethersulfone film.

1-1. Porous polyimide film

Polyimide is a general term for polymers containing imide bonds in the repeating unit, and usually it refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

The porous polyimide film which can be used for the invention is preferably a porous polyimide film including (as a main component) a polyimide obtained from a tetracarboxylic dianhydride and a diamine, and more preferably, it is a porous polyimide film comprising a polyimide obtained from a tetracarboxylic dianhydride and a diamine. The phrase "including as the main component" means that it essentially contains no components other than the polyimide obtained from a tetracarboxylic dianhydride and a diamine, as constituent components of the porous polyimide film, or that it may contain them but they are additional components that do not affect the properties of the polyimide obtained from the tetracarboxylic dianhydride and diamine.

In an embodiment, the porous polyimide film which may be used for the present invention also includes colored porous polyimide films obtained by forming a polyamic acid solution composition containing a polyamic acid solution obtained from a tetracarboxylic acid component and a diamine component, and a coloring precursor, and then heat treating it at 250°C or higher.

A polyamic acid is obtained by polymerization of a tetracarboxylic acid component and a diamine component. A polyamic acid is a polyimide precursor that can be cyclized to a polyimide by thermal imidization or chemical imidization.

The polyamic acid used may be any one that does not have an effect on the invention, even if a portion of the amic acid is imidized. Specifically, the polyamic acid may be partially thermally imidized or chemically imidized.

When the polyamic acid is to be thermally imidized, there may be added to the polyamic acid solution, if necessary, an imidization catalyst, an organic phosphorus-containing compound, or fine particles such as inorganic fine particles or organic fine particles. Also, when the polyamic acid is to be chemically imidized, there may be added to the polyamic acid solution, if necessary, a chemical imidization agent, a dehydrating agent, or fine particles such as inorganic fine particles or organic fine particles. Even if such components are added to the polyamic acid solution, they are preferably added under conditions that do not cause precipitation of the coloring precursor.

In this specification, a "coloring precursor" is a precursor that generates a colored substance by partial or total carbonization under heat treatment at 250°C or higher.

Coloring precursors usable for the production of the porous polyimide film are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C or higher, preferably 260°C or higher, even more preferably 280°C or higher and more preferably 300°C or higher, for carbonization to produce a colored substance, more preferably producing a black colored substance, with carbon-based coloring precursors being more preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

Moreover, in another embodiment, examples of the porous polyimide film which may be used for the preset invention also include a porous polyimide film which can be obtained by molding a polyamic acid solution derived from a tetracarboxylic acid component and a diamine component followed by heat treatment without using the coloring precursor.

The porous polyimide film produced without using the coloring precursor may be produced, for example, by casting a polyamic acid solution into a film, the polyamic acid solution being composed of 3 to 60% by mass of polyamic acid having an intrinsic viscosity number of 1.0 to 3.0 and 40 to 97% by mass of an organic polar solvent, immersing or contacting in a coagulating solvent containing water as an essential component, and imidating the porous film of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, at least one surface of the resulting porous polyimide film may be subjected to plasma treatment.

The tetracarboxylic dianhydride which may be used for the production of the porous polyimide film may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Also, preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine which may be used for the production of the porous polyimide film, any diamine may be used. Specific examples of diamines include the following:

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl]ether, bis[3-(4-aminophenoxy)phenyl]ether, bis[4-(3-aminophenoxy)phenyl]ether, bis[4-(4-aminophenoxy)phenyl]ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl] sulfide, bis[3-(4-aminophenoxy)phenyl] sulfide, bis[4-(3-aminophenoxy)phenyl] sulfide, bis[4-(4-aminophenoxy)phenyl] sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis[4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly, at least one type of diamine selected from the group consisting of benzenediamines, diaminodiphenyl ethers and bis(aminophenoxy)phenyl is preferred.

From the viewpoint of heat resistance and dimensional stability under high temperature, the porous polyimide film which may be used for the invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the porous polyimide film which can be used for the invention is preferably a porous polyimide film comprising one of the following aromatic polyimides.

(i) an aromatic polyimide comprising at least one tetracarboxylic acid unit selected from the group consisting of biphenyltetracarboxylic acid units and pyromellitic acid units, and an aromatic diamine unit,
(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units,
and/or,
(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The porous polyimide film used for the present invention is preferably a porous polyimide film which includes a three-layer structure porous polyimide film having a surface layer A and a surface layer B, the surface layers having a plurality of pores, and a macrovoid layer sandwiched between the surface layers A and B; wherein the average pore diameter of the pore present on the surface layer A is 0.01 $\mu$m to 15 $\mu$m, and the average pore diameter of the pore present on the surface layer B is 20 $\mu$m to 100 $\mu$m; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 $\mu$m; wherein the pores on the surface layers A and B communicate with the macrovoid, the total film thickness is 5 to 500 $\mu$m, and the porosity is 40% or more and less than 95%. In this case, at least one partition wall in the macrovoid layer has one or two or more pores connecting the neighboring macrovoids and having the average pore diameter of 0.01 to 100 $\mu$m, preferably 0.01 to 50 $\mu$m.

For example, porous polyimide films described in WO2010/038873, Japanese Unexamined Patent Publication (Kokai) No. 2011-219585 or Japanese Unexamined Patent Publication (Kokai) No. 2011-219586 can be used for the present invention.

1-2. Porous polyethersulfone film (Porous PES film)

The porous polyethersulfone film which may be used for the present invention contains polyethersulfone and typically consists substantially of polyethersulfone. Polyethersulfone may be synthesized by the method known to those skilled in the art. For example, it may be produced by a method wherein a dihydric phenol, an alkaline metal compound and a dihalogenodiphenyl compound are subjected to polycondensation reaction in an organic polar solvent, a method wherein an alkaline metal di-salt of a dihydric phenol previously synthesized is subjected to polycondensation reaction with dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the porous polyethersulfone membrane; and it is preferably 1.0 or less, more preferably 0.9 or less, further preferably 0.8 or less, particularly preferably 0.75 or less from the viewpoint of the easy production of a porous polyethersulfone film.

Further, from the viewpoints of heat resistance and dimensional stability under high temperature, it is preferred that the porous polyethersulfone film or polyethersulfone as a raw material thereof has a glass transition temperature of 200°C or higher, or that a distinct glass transition temperature is not observed.

The method for producing the porous polyethersulfone film which may be used for the present invention is not particularly limited. For example, the film may be produced by a method including the following steps:

a step in which polyethersulfone solution containing 0.3 to 60% by mass of polyethersulfone having logarithmic viscosity of 0.5 to 1.0 and 40 to 99.7% by mass of an organic polar solvent is casted into a film, immersed in or contacted with a coagulating solvent containing a poor solvent or non-solvent of polyethersulfone to produce a coagulated film having pores; and

a step in which the coagulated film having pores obtained in the above-mentioned step is heat-treated for coarsening of the aforementioned pores to obtain a porous polyethersulfone film;

wherein the heat treatment includes the temperature of the coagulated film having the pores is raised higher than the glass transition temperature of the polyethersulfone, or up to 240°C or higher.

The porous polyethersulfone film which can be used in the present invention is preferably a porous polyethersulfone film having a surface layer A, a surface layer B, and a macrovoid layer sandwiched between the surface layers A and B,

wherein the macrovoid layer has a partition wall bonded to the surface layers A and B, and a plurality of macrovoids surrounded by such a partition wall and the surface layers A and B, the macrovoids having the average pore diameter in the planar direction of the film of 10 to 500 $\mu$m;

wherein the thickness of the macrovoid layer is 0.1 to 50 $\mu$m,

each of the surface layers A and B has a thickness of 0.1 to 50 $\mu$m,

wherein one of the surface layers A and B has a plurality of pores having the average pore diameter of more than 5 $\mu$m and 200 $\mu$m or less, while the other has a plurality of pores having the average pore diameter of 0.01 $\mu$m or more and less than 200 $\mu$m,

wherein one of the surface layers A and B has a surface aperture ratio of 15% or more while other has a surface aperture ratio of 10% or more,

wherein the pores of the surface layers A and B communicate with the macrovoids,

wherein the porous polyethersulfone film has total film thickness of 5 to 500 $\mu$m and a porosity of 50 to 95%.

Since the aforementioned porous polymer film as a cell culture carrier which may be used for the cell culture apparatus of the present invention has a low hydrophilic porous property, liquid is stably held in the porous polymer film, and a wet environment is maintained that is also resistant to drying. It is therefore possible to achieve survival and proliferation of cells even in very small amounts of medium compared with a cell culturing apparatus using the conventional cell culture carrier. Furthermore, since it is possible to carry out culturing even if some or all of the porous polymer film has been exposed to air, oxygen can be efficiently supplied to the cells, and mass culturing of cells is made possible.

According to the invention, the amount of medium used is extremely minimal, and the porous polymer film as the culture support can be exposed to a gas phase, thereby allowing oxygen supply to the cells to be adequately accomplished by diffusion. According to the invention, therefore, there is no particular need for an oxygen supply apparatus.

2. Cell culture apparatus

An embodiment of the present invention relates to a cell culture apparatus comprising:

a porous polymer film;

a cell culture section containing the porous polymer film;

a medium supplying means disposed at an upper portion of the cell culture section; and

a medium collecting means disposed at a lower portion of the cell culture section, wherein the porous polymer film is a three-layer structure porous polymer film having a surface layer A and a surface layer B, the surface layers having a plurality of pores, and a macrovoid layer sandwiched between the surface layers A and B; wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B;

wherein the cell culture section comprises a bottom portion having one or more medium discharge port(s), and a side portion disposed approximately perpendicular to the bottom portion. The cell culture apparatus will be hereinafter also referred to as a "cell culture module of the invention". The embodiment of the cell culture apparatus of the present invention will be illustrated with reference to the drawings.

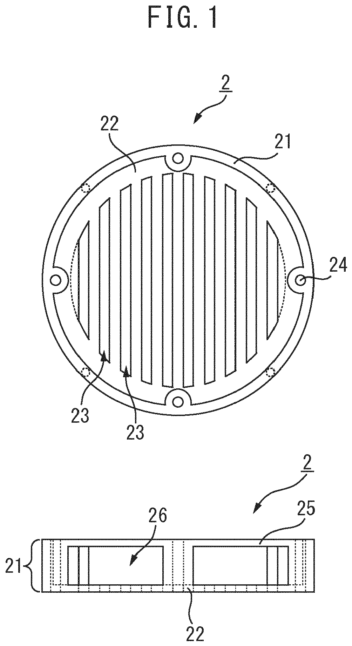

FIG. 1 is a diagram illustrating a cell culture section 2 which constitutes a cell culture apparatus of the invention. The cell culture section 2 has a bottom section 22 to mount the aforementioned porous polymer film thereon, and a side section 21 disposed approximately vertical to the bottom section 22. The bottom section 22 is provided with one or more medium discharge port 23 to discharge a medium being dropped from the medium supplying means 3 described later. The shape or number of the medium discharge port 23 is not particularly limited so long as possessing a function to discharge a medium to a cell culture section 2 on the lower stage or medium collecting means 4 (described later) without the porous polymer film being detached. In this example, a medium discharge port 23 like a slit is provided. The side section 21 is further provided with one or more medium supplying ports 26 so that a medium is supplied from the lateral direction of the cell culture unit 2. The position and size of the medium supplying port 26 may be appropriately altered as appropriate according to the purpose. In this embodiment, an appearance of the cell culture unit 2 is cylindrical, but is not limited thereto, and may be in an arbitrary form such as a triangular prism, or a rectangular column. However, since the cell culture unit 2 may be stacked to be used as described later, the upper face and a lower face (bottom face 22) of the cell culture unit 2 is preferably in the same shape and parallel.

In this specification, a "medium" refers to a cell culture medium for culturing cells, especially animal cells. The term "medium" is interchangeably used as "cell culture medium". Accordingly, the medium used in the invention refers to a liquid medium. As for types of a medium, the ordinarily used medium may be used and appropriately determined depending on the types of cells to be cultured.

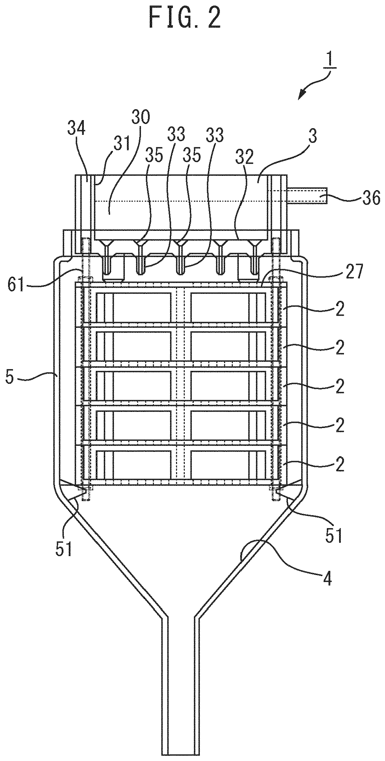

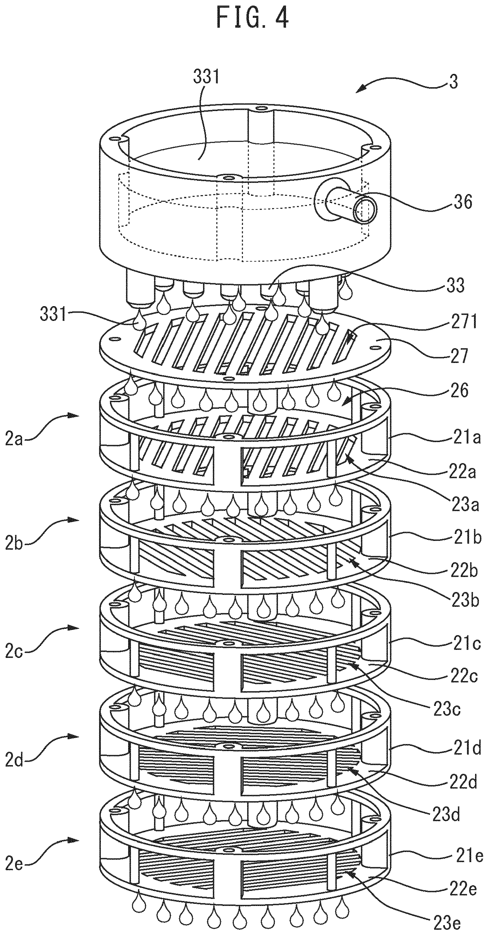

FIG. 2 is a diagram illustrating a configurational example of a cell culture apparatus of the invention. Five stages of the cell culture unit 2 are stacked, and a lid section 27 is mounted on the top stage of the cell culture unit 2. In the lid section 27, a plurality of slit-like medium discharge ports 271 (see, FIG. 4) are provided, like a medium discharge port 23 provided in the bottom section 22. The shape or number of the medium discharge port 271 is not particularly limited so long as possessing a function to discharge a medium to a cell culture section 2 on the lower stage.

In a cell culture section 2 on each stage, the aforementioned porous polymer film is contained. The lowest stage of the stacked cell culture unit 2 is mounted on a stopper 51 provided inside an outer cylinder 5. In this embodiment, an outer cylinder 5 is formed of a cylindrical section to contain a stacked cell culture unit 2, and a funnel-like-shaped medium collecting means 4 to collect a medium dropped through each stage of a cell culture unit 2. In this embodiment, a medium collecting means 4 is contained in a part of the outer cylinder 5. The medium collecting means 4 is not particularly limited so long as having a shape capable of collecting the medium dropped from the upper section. However, from a viewpoint of efficiently aggregating cells, it is preferably of a funnel-like shape. The shape of the outer cylinder 5 may be appropriately altered depending on the shape of the aforementioned cell culture unit 2. On the top stage of the cell culture unit 2, a medium supplying means 3 is placed. Positions of a fastener inserting port 24 provided in the cell culture unit 2 and a fastener inserting port 34 provided in the medium supplying means 3 were adjusted so that they were perpendicular to each other, and a fastener 61 was inserted from a fastener inserting port 34 to place a medium supplying means 3 and a cell culture unit 2 at appropriate positions.

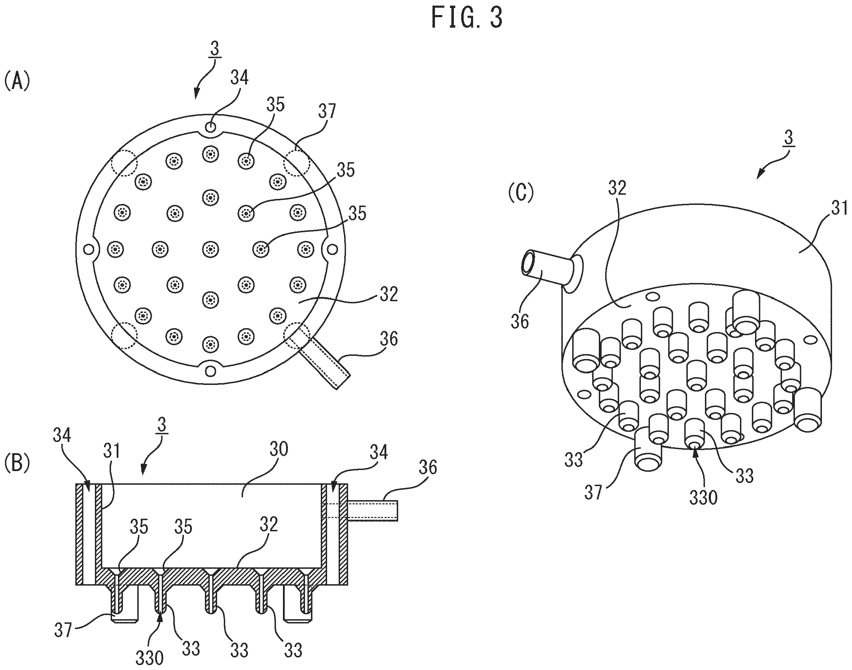

FIG 3 is a diagram illustrating a medium supplying means 3. The medium supplying means 3 illustrated in FIG. 3 is provided with a medium storage unit 30 of cells formed of a side section 31 and a bottom section 32. The bottom section 32 is provided with a plurality of medium dropping ports 35, and the medium dropping port 35 is tapered toward a center. In addition, at the bottom section 32, on the other side (outer side) of the medium storage unit 30, a medium dropping nozzle 33 communicating with a medium dropping pore 35 is formed. A medium dropping nozzle 33 has, at the center thereof, a nozzle port 330 communicating with the medium dropping port 35. A medium stored in a medium storage unit 30 is passed through a medium dropping port 35, transferred through a nozzle port 330 of the medium dropping nozzle 33. In this way, a predetermined amount is dropped. A diameter of the medium dropping port 35, an angle of the tapered shape, a shape of the tip of the medium dropping nozzle 33, a diameter of the nozzle port 330 and the like may be appropriately adjusted to adjust the amount of a medium dropped from the nozzle port 330 and the dropping speed. At the bottom section 32, the medium dropping port 35 is placed concentrically from a center of a circle and equally spaced. In this way, a medium is equally dropped from a medium dropping nozzle 33.

An overflow pipe 36 is provided at an arbitrary position of a side section 31 of a medium supplying means 3. The overflow pipe 36 prevents a medium from overflowing a medium storage unit 30 and flowing out along a side section 31. The position of the overflow pipe 36 determines an amount of a medium to be contained in the medium storage unit 30. A leg section 37 is provided on the same side as a medium dropping nozzle 33 of bottom section 32. The length of the leg section is longer than that of the medium dropping nozzle 33. By adjusting the length of the leg section 37, the position of the medium to be supplied to a cell culture unit 2 can be determined. At least three leg sections 37 having the same length are installed, thereby placing a medium supplying means 3 at the upper part of the cell culture unit 2. The leg section 37 might not be installed. When the leg section 37 is not installed, it may be, for example, fitted in the upper part of the outer cylinder 5.

FIG. 4 is a perspective view illustrating an exemplary configuration of the cell culture apparatus 1 of the invention, in which a cell culture unit 2 and a medium supplying means 3 from the exemplary configuration in FIG. 2 are respectively depicted independently in the vertical direction. In this embodiment, slit-like medium discharge ports 23a are provided parallel to each other in a bottom section 22a of the cell culture unit 2a while slit-like medium discharge ports 23b are provided parallel to each other in a bottom section 22b of the cell culture unit 2b which is one stage lower than 2a, with the ports 23b being provided at the position rotated in counterclockwise direction by 30 degree. In the same manner, medium discharge ports 23c to 23e are provided so that the position of the medium discharge ports 23 in the upper stage is rotated by 30 degree to the position of the medium discharge ports 23 in the lower stage. In this way, a medium is dropped efficiently from the upper stage to the lower stage in a multi-layered cell culture unit.

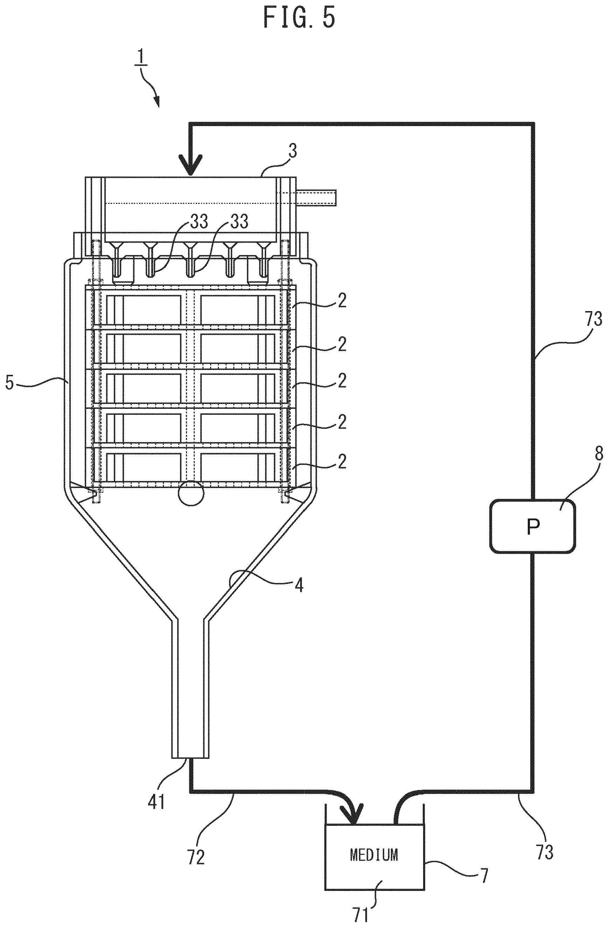

FIG. 5 is a diagram illustrating a configurational example of a cell culture apparatus 1 of the present invention. A medium collecting means 4 which is a part of the outer cylinder 5 is funnel-like, and the dropped medium is aggregated in a medium discharging section 41. A medium discharging section 41 communicates with one end section of the medium discharging line 72, while the other end section of the medium discharging line 72 communicates with a medium storage tank 7. A medium storage tank 7 communicates with one end section of the medium supplying line 73, while the other end section of the medium supplying line 73 communicates with a medium supplying means 3. In this way, the medium discharged from the cell culture apparatus 1 is stored in the medium storage tank 7, and the stored medium is supplied again into the cell culture apparatus 1, and thus the medium is circulated. By circulating a medium, it is possible to increase a concentration of a protein produced by cells supported on the porous polymer film, thereby enhancing a protein collecting efficiency. It is also possible to supply a fresh medium by periodically exchanging a medium 71 discharged into the cell storage tank 7. Although not illustrated here, a separate medium storage tank may be provided which supplies a fresh medium. In this case, the medium storage tank 7 stores only a used medium, and a fresh medium is supplied from the medium storage tank through the medium supplying line 73 to the cell supplying means 3. A pump 8 for pumping a medium is provided in the middle of the medium supplying line 73. The type of the medium supply pump 8 is not particularly limited. For example, a peristaltic pump or the like may be used.

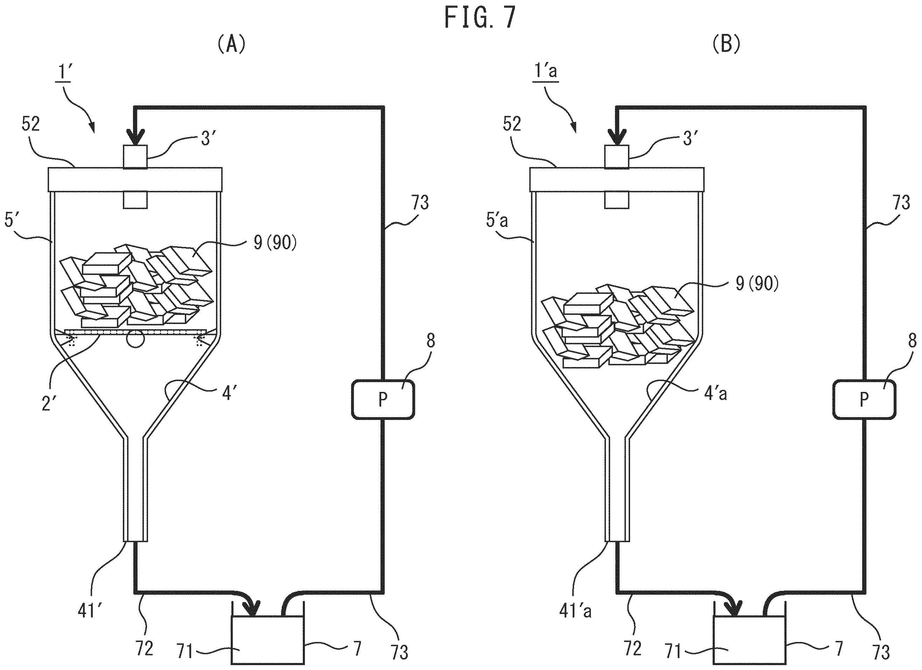

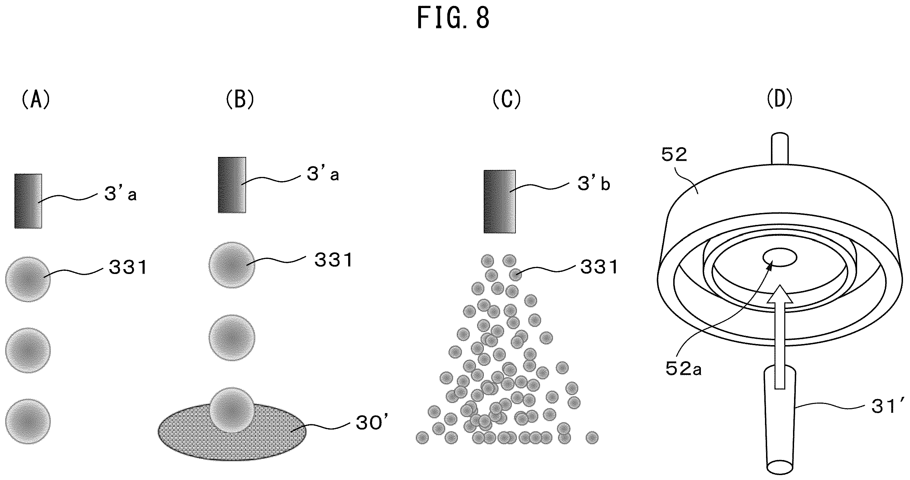

In another embodiment of the present invention, a medium supplying means 3 may be a medium droplet supplying means (for example, a medium droplet supplying means 3' in FIG. 7) which supplies a medium as a dropletized medium. In this specification, a "dropletized medium" means a mist-like or dropletized medium (for example, as in Fig. 8(C)), which is a medium ready for jetting or spraying onto a porous polymer film used for the present invention. The diameter of the dropletized medium is not limited but may be a fine mist-like dropletized medium so as to be floatable in an air without being freely fallen by the gravity. The diameter of the mist-like dropletized medium may be about 1 $\mu$m to 100 $\mu$m, or may be even smaller. In addition, a dropletized medium may be, for example, droplet-like medium which freely falls by the gravity, for example more than 100 $\mu$m. Examples of a method for forming droplet of a medium include, for example, a method for forming droplet by a known means. For example, a medium may be dropletized using a mist-like nozzle, a shower-like nozzle or the like. However, a method for dropletizing is preferably a method which does not alter components of the medium. For example, a method in which the medium is evaporated is excluded from the droplet forming methods.

In an embodiment of the present invention, a dropletized medium is supplied to a porous polymer film with a cell supported thereon. A dropletized medium is passed through a gas phase before it reaches to a porous polymer film. Thus, oxygen is dissolved in a medium. In this way, a medium having a sufficient amount of oxygen is continuously supplied, and it is possible to perform culture without cells becoming ischemic. In addition, since a porous polymer film is always exposed to a gas phase, it is possible for a medium adhering to the porous polymer film to always intake oxygen, enabling culture while efficiently supplying oxygen.

A medium droplet supplying means may be placed in the upper part of the cell culture unit 2, or may be placed on the side face of the cell culture unit 2, or may be placed in the lower part of the cell culture unit 2. A plurality of the medium droplet supplying means may be provided. In an embodiment of the present invention, a medium droplet supplying means may be provided in a sealed outer cylinder 5. In this way, the inside of the outer cylinder 5 is filled with a mist-like medium, which means a medium is supplied homogeneously to a porous polymer film having cells supported thereon.

Another embodiment of the present invention relates to a cell culture apparatus comprising:

> a porous polymer film;
> a cell culture section containing the porous polymer film;
> a medium supplying means disposed at a upper portion of the cell culture section; and
> a medium collecting means disposed at a lower portion of the cell culture section,

wherein the porous polymer films are a three-layer structure porous polymer film having a surface layer A and a surface layer B, the surface layers having a plurality of pores, and a macrovoid layer sandwiched between the surface layers A and B;

wherein an average pore diameter of the pores present in the surface layer A is smaller than an average pore diameter of the pores present in the surface layer B;

wherein the macrovoid layer has a partition wall bonded to the surface layers A and B, and a plurality of macrovoids surrounded by such a partition wall and the surface layers A and B;

wherein the pores in the surface layers A and B communicate with the macrovoid; and

wherein the medium collecting means is a part of an outer cylinder for housing the cell culture section.

As illustrated in FIG. 7(A), in a cell culture apparatus 1' of an embodiment of the invention, a medium collecting means 4' is a part of the outer cylinder 5' in which the dropped medium is aggregated to a medium discharging section 41'. In addition, a porous polymer film 9 is placed in the outer cylinder 5', and a cell culture unit 2' to be housed in the outer cylinder 5' is provided. The cell culture unit 2' is provided with one or more medium discharge ports to discharge the medium dropped from the medium droplet supplying means 3'. The shape and number of the medium discharge port is not particularly limited so long as having a function to discharge a porous polymer film to the medium collecting means 4' without the porous polymer film being detached. For example, a slit-like, mesh-like, or a small pore medium discharge port is provided.

As illustrated in FIG. 7(B), in a cell culture apparatus 1'a according to another embodiment of the invention, a medium collecting means 4' may be a cell culture unit/medium collecting means 4'a which has a function of the cell culture unit 2' as well. In this case, a porous polymer film 9 may be directly placed on the cell culture unit/medium collecting means 4'a. It is preferred to place a modularized porous polymer film 90 described later is placed on the cell culture unit/medium collecting means 4'a. By using the modularized porous polymer film, an arbitrary number of modularized porous polymer may be easily contained on the cell culture unit/medium collecting means 4'a.

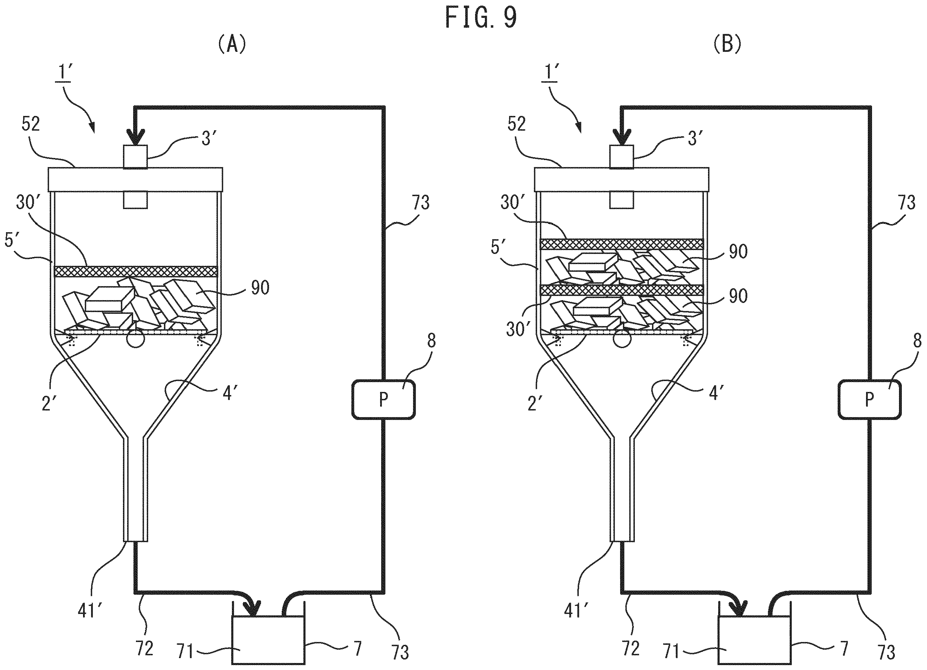

For example, droplets 331, such as drop-type droplets as in FIG. 8(A) or shower-type droplets as in FIG. 8(C) are supplied from the medium droplet supplying means 3'. The shape of a droplet 331 may be altered by appropriately selecting a known nozzle as a medium droplet supplying means 3'. In addition, as in FIG. 8(B), droplets 331 released from the medium droplet supplying means 3'a may be further applied to a droplet mesh 30' to homogeneously supply the droplets to the whole porous polymer film. As the droplet mesh 30', a mesh made of, for example but not limited thereto, polystyrene, polycarbonate, polymethyl methacrylate, polyethylene terephthalate, stainless steel may be used. In another embodiment, the droplet mesh 30', for example, as in FIG. 9(A), may be provided in the lower part of the medium droplet supplying means 3'. In another embodiment, as in FIG. 9(B), the droplet mesh 30' may be used between the porous polymer films and/or modularized porous polymer films to be multi-layered. The mesh structure of the droplet mesh 30' may have a mesh opening such that the medium droplet is diffused entirely over the droplet mesh 30' upon application to supply the medium to the lower face side of the droplet mesh 30'. For example, a stainless steel mesh wound into a mesh bundle 31' may be used as an embodiment of the medium droplet supplying means 3'. As illustrated in FIG. 8(D), the mesh bundle 31' is inserted into a lid section medium supplying port 52a provided on the inner side of the outer cylinder lid section 52. In this way, a medium may be dropped directly onto the porous polymer film without the medium falling along the inner wall of the outer cylinder. In addition, joint of the outer cylinder and the outer cylinder lid section may be protected from contamination. In this embodiment, a mesh bundle 31' made of stainless steel is used. However, the configuration which exhibits a function to drop a medium directly onto a porous polymer film without falling along an inner wall of an outer cylinder may be used without limited to the stainless steel mesh. In addition, as the droplet mesh 31', a mesh made of, for example but not limited thereto, polystyrene, polycarbonate, polymethyl methacrylate, polyethylene terephthalate, stainless steel may be used.

The porous polymer film used in the present embodiment may be applied onto the bottom section 22 of the cell culture unit 2 with:

i) being folded up;
ii) being wound into a roll-like shape;
iii) sheets or pieces thereof being concatenated with a thread-like structure; and/or
iv) being tied together into a rope-like shape.

The porous polymer film used in the present embodiment may be applied onto the bottom section 22 of the cell culture unit 2 with: v) two or more of them being stacked. By forming into shapes such as i) to v), it is possible to place a large amount of porous polymer films into a fixed volume of cell culture medium.

As the porous polymer film used in the present embodiment, a modularized porous polymer film (hereinafter referred to as a "modularized porous polymer film") may be used. In this specification, "a modularized porous polymer film" means a porous polymer film contained in a casing. It should be noted that the phrase "a modularized porous polymer film" may be expressed simply as "a module", both expressions can be used interchangeably to indicate the same meaning.

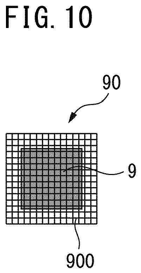

A casing which is contained in a modularized porous polymer film used in the embodiment of the present invention has two or more cell culture medium flow inlets and such cell culture medium flow inlet makes the medium to flow in or out of the casing. The diameter of the cell culture medium flow inlet of the casing is preferably larger than the diameter of the cell so as to enable cell to flow into the casing. In addition, the diameter of the cell culture medium flow inlet is preferably smaller than the diameter of the cell which porous polymer film flows out through the cell culture medium flow inlet. The diameter smaller than the diameter of the porous polymer film which flows out may be appropriately selected depending on the shape and size of the porous polymer film contained in the casing. For example, in the case where the porous polymer film has string-like shape, the diameter is not particularly limited so long as it is smaller than the width of the shorter side of the porous polymer film so that the porous polymer film is prevented from flowing out. As for the number of the cell culture medium flow inlets, it is preferred to provide as many culture medium flow inlets as possible so that the cell culture medium may be easily supplied into and/or discharged out of the casing. It is preferably 5 or more, preferably 10 or more, preferably 20 or more, preferably 50 or more, and preferably 100 or more. As the cell culture medium flow inlet, the casing may have a mesh-like structure in part or in whole. Moreover, the casing itself may be mesh-like. In the present invention, examples of mesh-like structure include, but not limited to, lattices including longitudinal, transverse, and/or oblique elements wherein individual apertures form cell culture medium flow inlet which allows the fluid to pass therethrough. For example, it has a structure illustrated in FIG. 10.

Examples of a casing of a modularized porous polymer film used in an embodiment of the present invention include, for example, polystyrene, polycarbonate, polymethyl methacrylate, polyethylene terephthalate; metals such as stainless steel, but not limited thereto, and are not particularly limited so long as they have no effect on cell culture.

The modularized porous polymer film used in the embodiment of the present invention is a modularized porous polymer film contained in the casing with:

(i) the two or more independent porous polymer films being aggregated;
(ii) the porous polymer films being folded up;
(iii) the porous polymer films being wound into a roll-like shape; and/or
(iv) the porous polymer film being tied together into a rope-like shape.

wherein it is possible to apply the modularized porous polymer film to the cell culture section 2.

In this specification, "two or more independent porous polymer films are aggregated and contained within a casing" means that two or more independent porous polymer films are aggregated and contained in a predetermined space surrounded by a casing. According to the present invention, the two or more independent porous polymer films may be immovably fixed by fixing at least one point of the porous polymer film to at least one point of the casing by an arbitrary method. In addition, the two or more independent porous polymer films may be fragments. A fragment may take an arbitrary shape such as circular, elliptical, quadrilateral, triangular, polygonal or string-like shape, and is preferably a string-like shape or quadrilateral shape. In the present invention, the fragments may be of any size. When it has string-like shape, it may be of any length, but, for example, preferably 80 mm or less, preferably 30 mm or less, and more preferably 10 mm or less. This can protect cells to be grown in the porous polymer film from stress to be applied. In addition, when the fragments of the porous polymer film are substantially square, it may be formed so that length of each side may match the inner wall or may be shorter than each side of the inner wall (e.g. shorter by about 0.1 mm to 1 mm), rendering the porous polymer film immovable in the casing. In the present invention, when the fragments have a substantially square shape, the side may be any length, but, for example, preferably 80 mm or less, preferably 50 mm or less, more preferably 30 mm or less, still more preferably 20 mm or less, and may be 10 mm or less.

In this specification, "the porous polymer films being folded up" means a porous polymer film which is folded up in the casing, and thus it is rendered immovable in the casing by frictional force between each surfaces of the porous polymer film and/or the inner surface of the casing. In this specification, "being folded up" may indicate the pours polymer film being creased or creaseless.

In this specification, "the porous polymer films being wound into a roll-like shape" means the porous polymer film being wound into a roll-like shape and thus it is rendered immovable in the casing by frictional force between each surfaces of the porous polymer film and/or the inner surface of the casing. Moreover, in the present invention, the porous polymer film being twisted together into a rope-like shape means, for example, more than one porous polymer films in rectangle strip shape are knitted into a rope-shape by arbitrary method, rendering the porous polymer films immovable by the mutual frictional force of the porous polymer films. It is also possible that (i) the two or more independent porous polymer films being aggregated; (ii) the porous polymer films being folded up; (iii) the porous polymer films being wound into a roll-like shape; and/or (iv) the porous polymer film being tied together into a rope-like shape may be combined and contained within a casing.

In this specification, "the porous polymer film being immovable in the casing" means that the porous polymer film is contained in the casing so that the porous polymer film is continually morphologically unchanged during culturing the cell culture module in the cell culture medium. In other words, the porous polymer film itself is in a suppressed state to be prevented from continual waving movement by fluid. Since the porous polymer film is kept immovable in the casing, the cell being grown in the porous polymer film is protected from stress to be applied, enabling stable cell culture without cells being killed by apoptosis.

3. Cell culture method using cell culture apparatus

<Step for applying cells to porous polymer film>

There are no particular restrictions on the specific steps for application of the cells usable for the present invention to the porous polymer film. It is possible to carry out the steps described throughout the present specification, or to employ any desired method suited for applying cells to a film-like support. Application of cells to the porous polymer film in the method of the invention includes, but is not limited to, the following embodiments.
(A) An embodiment including a step of seeding cells on the surface of a porous polymer film;
(B) An embodiment including steps of placing a cell suspension on the dried surface of a porous polymer film; allowing it to stand, or moving the porous polymer film to promote efflux of the liquid, or stimulating part of the surface to cause absorption of the cell suspension into the film, and
retaining the cells in the cell suspension inside the film and allowing the water to flow out;
and
(C) An embodiment including a step of:

wetting one or both sides of a porous polyimide film with a cell culture medium or a sterilized liquid, loading a cell suspension into the wetted porous polyimide film, and
retaining the cells in the cell suspension inside the film and allowing the water to flow out.

Embodiment (A) includes a step of directly seeding cells or a cell mass on the surface of a porous polymer film. Alternatively, it includes an embodiment of placing a porous polyimide film in a cell suspension and wetting the porous polyimide film from the surface thereof with the cell culture medium.
Cells seeded on the surface of a porous polymer film adhere to the porous polymer film and infiltrate into the interiors of the pores. Preferably, the cells adhere to the porous polymer film without applying any particular exterior physical or chemical force. The cells that have been seeded on the surface of the porous polymer film can stably grow and proliferate on the surface and/or in the interior of the film. The cells may be in a variety of different forms, depending on the location of the film used for growth and proliferation.
For Embodiment (B), a cell suspension is placed on the dried surface of a porous polymer film. The porous polymer film is allowed to stand, or the porous polymer film is moved to promote efflux of the liquid, or part of the surface is stimulated to cause absorption of the cell suspension into the film, so that the cell suspension permeates into the film. While it is not our intention to be constrained by theory, this is believed to be due to the properties of each of the surface forms of the porous polymer film. According to this embodiment, the cells are absorbed and seeded in the locations of the film where the cell suspension has been loaded.
Alternatively, as according to Embodiment (C), after all or a portion of one or both sides of the porous polymer film has been wetted with the cell culture medium or sterilized liquid, the cell suspension may be loaded into the wetted porous polymer film. This will significantly increase the transit rate of the cell suspension.
For example, a method of wetting a portion of the film edges, for the main purpose of preventing fly loss of the film, may be used (hereunder referred to as "single-point wetting method"). The single-point wetting method is nearly the same as the dry method (Embodiment (B)) in which the film essentially is not wetted. However, it is possible that cell solution permeation through the film is more rapid at the small wetted portions. There may also be used a method in which all of one or both sides of the porous polymer film that have been thoroughly wetted (hereunder this will also be referred to as "wet film") is loaded with a cell suspension (this will hereunder be referred to as "wet film method"). In this case, the entire porous polymer film has a greatly increased transit rate for the cell suspension.
According to Embodiments (B) and (C), the cells in the cell suspension are retained in the film, while the water flows out. This allows treatment such as increasing the concentration of cells in the cell suspension and flowing out of unwanted non-cellular components together with the water.
Embodiment (A) will also be referred to as "natural seeding", and Embodiments (B) and (C) as "suction seeding".
Preferably, but not restrictively, the viable cells are selectively retained in the porous polymer film. Thus, according to a preferred embodiment of the invention, the viable cells are retained in the aforementioned porous polymer film, and the dead cells preferentially flow out together with the water.
The sterilized liquid used for Embodiment (C) is not particularly restricted, and may be a sterilized buffering solution or sterilized water. A buffering solution may be, for example, (+) or (-) Dulbecco's PBS, or (+) or (-) Hank's Balanced Salt Solution. Examples of buffering solutions are listed in Table 1 below.

[Table 1]

| Component | Concentration (mmol/L) | Concentration (g/L) |
|---|---|---|
| NaCl | 137 | 8.00 |
| KCl | 2.7 | 0.20 |
| $Na_2HPO_4$ | 10 | 1.44 |
| $KH_2PO_4$ | 1.76 | 0.24 |
| pH(-) | 7.4 | 7.4 |

In the method of the invention, application of cells to the porous polymer film further includes an embodiment of making adherent cells in a floating state as a suspension to coexist with the porous polymer film, to adhere the cells with the film (entangling). For example, for application of the cells to the porous polymer film in the cell culturing method of the invention, the cell culture medium, the cells and one or more of the porous polymer films may be placed in the cell culturing vessel. When the cell culture medium is a liquid, the porous polymer film is in a floating state in the cell culture medium. The cells can adhere to the porous polymer film due to the properties of the porous polymer film. Thus, even with cells that are not suited for natural suspension culture, the porous polymer film allows culturing in a floating state in the cell culture medium. The cells preferably adhere to the porous polymer film. Here, "adhere spontaneously" means that the cells are retained on the surface or in the interior of the porous polyimide film without applying any particular exterior physical or chemical force.

Two or even more forms of the methods of the invention may be used in combination to apply the aforementioned cells to a porous polymer film. For example, two or more of the Embodiments (A) to (C) may be combined to apply cells to a porous polymer film. It is possible to conduct culture by apply a porous polymer film having cells supported thereon to a cell culture unit 2 in the aforementioned cell culture apparatus 1.

In addition, a medium containing a suspended cell may be previously added dropwise and seeded to a cell culture unit 2 in which a porous polymer film is contained from a cell supplying means 3.

In this specification, a "suspended cell" encompasses cells obtained by forcing to suspend an adherent cell in a medium with a proteolytic enzyme such as trypsin, and cells which can be suspension-cultured in a medium, obtained by the aforementioned conditioning step, and etc..

The types of the cells which may be used for the present invention may be selected from the group consisting of animal cells, insect cells, plant cells, yeast cells and bacteria. Animal cells are largely divided into cells from animals belonging to the subphylum Vertebrata, and cells from non-vertebrates (animals other than animals belonging to the subphylum Vertebrata). There are no particular restrictions on the source of the animal cells, for the purpose of the present specification. Preferably, they are cells from an animal belonging to the subphylum Vertebrata. The subphylum Vertebrata includes the superclass Agnatha and the superclass Gnathostomata, the superclass Gnathostomata including the class Mammalia, the class Aves, the class Amphibia and the class Reptilia. Preferably, they are cells from an animal belonging to the class Mammalia, generally known as mammals. Mammals are not particularly restricted but include, preferably, mice, rats, humans, monkeys, pigs, dogs, sheep and goats.

The types of animal cells or plant cells that may be used for the invention are not particularly restricted, but are preferably selected from the group consisting of pluripotent stem cells, tissue stem cells, somatic cells and germ cells.

The term "pluripotent stem cells", in this specification, is intended as a comprehensive term for stem cells having the ability to differentiate into cells of any tissues (pluripotent differentiating power). While not restrictive, pluripotent stem cells include embryonic stem cells (ES cells), induced pluripotent stem cells (iPS cells), embryonic germ cells (EG cells) and germ stem cells (GS cells). They are preferably ES cells or iPS cells. Particularly preferred are iPS cells, which are free of ethical problems, for example. The pluripotent stem cells used may be any publicly known ones, and for example, the pluripotent stem cells described in WO2009/123349 (PCT/JP2009/057041) may be used.

The term "tissue stem cells" refers to stem cells that are cell lines capable of differentiation but only to limited specific tissues, though having the ability to differentiate into a variety of cell types (pluripotent differentiating power). For example, hematopoietic stem cells in the bone marrow are the source of blood cells, while neural stem cells differentiate into neurons. Additional types include hepatic stem cells from which the liver is formed and skin stem cells that form skin tissue. Preferably, the tissue stem cells are selected from among mesenchymal stem cells, hepatic stem cells, pancreatic stem cells, neural stem cells, skin stem cells and hematopoietic stem cells.

The term "somatic cells" refers to cells other than germ cells, among the cells composing a multicellular organism. In sexual reproduction, these are not passed on to the next generation. Preferably, the somatic cells are selected from among hepatocytes, pancreatic cells, muscle cells, bone cells, osteoblasts, osteoclasts, chondrocytes, adipocytes, skin cells, fibroblasts, pancreatic cells, renal cells and lung cells, or blood cells such as lymphocytes, erythrocytes, leukocytes, monocytes, macrophages or megakaryocytes.

The term "germ cells" refers to cells having the role of passing on genetic information to the succeeding generation in reproduction. These include, for example, gametes for sexual reproduction, i.e. the ova, egg cells, sperm, sperm cells, and spores for asexual reproduction.

The cells may also be selected from the group consisting of sarcoma cells, established cell lines and transformants. The term "sarcoma" refers to cancer occurring in non-epithelial cell-derived connective tissue cells, such as the bone, cartilage, fat, muscle or blood, and includes soft tissue sarcomas, malignant bone tumors and the like. Sarcoma cells are cells derived from sarcoma. The term "established cell line" refers to cultured cells that are maintained in vitro for long periods and reach a stabilized character and can be semi-permanently subcultured. Cell lines derived from various tissues of various species including humans exist, such as PC12 cells (from rat adrenal medulla), CHO cells (from Chinese hamster ovary), HEK293 cells (from human embryonic kidney), HL-60 cells (from human leukocytes) and HeLa cells (from human cervical cancer), Vero cells (from African green monkey kidney epithelial cells), MDCK cells (from canine renal tubular epithelial cells), HepG2 cells (from human hepatic cancer), BHK cells (newborn hamster kidney cell), NIH3T3 cells (from mouse fetal fibroblast cells). The term "transformants" refers to cells with an altered genetic nature by extracellularly introduced nucleic acid (DNA and the like).

In this specification, an "adherent cell" is generally a cell which is required to adhere itself on an appropriate surface for growth, and is also referred to as an attachment cell or an anchorage-dependent cell. In certain embodiments of the present invention, the cells used are adherent cells. The cells used for the present invention are adherent cells, more preferably cells which may be cultured even as a suspension in a medium. The adherent cells which can be suspension cultured may be obtained by conditioning the adherent cells to a state suitable for suspension culture, and include, for example, CHO cells, HEK293 cells, Vero cells, NIH3T3 cells, and cell lines derived from these cells.

FIG. 1 represents a model diagram of cell culturing using a porous polymer film. FIG. 1 serves merely for illustration and the elements are not drawn to their actual dimensions. In the cell culture method of the invention, application of cells and culturing are carried out on a porous polymer film, thereby allowing culturing of large volumes of cells to be accomplished since large numbers of cells grow on the multisided connected pore sections on the inside, and the surfaces on the porous polymer film. Moreover, in the cell culture method of the invention, it is possible to culture large volumes of cells while drastically reducing the amount of medium used for cell culturing compared to the prior art. For example, large volumes of cells can be cultured even when all or a portion of the porous polymer film is not in contact with the liquid phase of the cell culture medium. In addition, the total volume of the cell culture medium in the cell culture vessel, with respect to the total porous polymer film volume including the cell survival zone, can be significantly reduced.

Throughout the present specification, the volume of the porous polymer film without cells, that occupies the space including the volume between the interior gaps, will be referred to as the "apparent porous polymer film volume" (see, FIG. 1). In the state where the cells are applied to the porous polymer film and the cells have been carried on the surface and the interior of the porous polymer film, the total volume of the porous polymer film, the cells and the medium that has wetted the porous polymer film interior, which is occupying the space therein, will be referred to as the "porous polymer film volume including the cell survival zone" (see, FIG. 1). When the porous polymer film has a film thickness of 25 μm, the porous polymer film volume including the cell survival zone is a value of at maximum about 50% larger than the apparent porous polymer film volume. In the method of the invention, a plurality of porous polymer films may be housed in a single cell culture vessel for culturing, in which case the total sum of the porous polymer film volume including the cell survival zone for each of the plurality of porous polymer films supporting the cells may be referred to simply as the "total sum of the porous polymer film volume including the cell survival zone".

Using the method of the invention, cells can be satisfactorily cultured for a long period of time even under conditions in which the total volume of the cell culture medium in the cell culture vessel is 10,000 times or less of the total sum of the porous polymer film volume including the cell survival zone. Moreover, cells can be satisfactorily cultured for a long period of time even under conditions in which the total volume of the cell culture medium in the cell culture vessel is 1,000 times or less of the total sum of the porous polymer film volume including the cell survival zone. In addition, cells can be satisfactorily cultured for a long period of time even under conditions in which the total volume of the cell culture medium in the cell culture vessel is 100 times or less of the total sum of the porous polymer film volume including the cell survival zone. In addition, cells can be satisfactorily cultured for a long period of time even under conditions in which the total volume of the cell culture medium in the cell culture vessel is 10 times or less of the total sum of the porous polymer film volume including the cell survival zone.

In other words, according to the invention, the space (vessel) used for cell culturing can be reduced to an absolute minimum, compared to a conventional cell culture apparatus for performing two-dimensional culture. Furthermore, when it is desired to increase the number of cells cultured, the cell culturing volume can be flexibly increased by a convenient procedure including increasing the number of layered porous polymer films. In a cell culture apparatus comprising a porous polymer film to be used for the invention, the space (vessel) in which cells are cultured and the space (vessel) in which the cell culture medium is stored can be separate, and the necessary amount of cell culture medium can be prepared according to the number of cells to be cultured. The space (vessel) in which the cell culture medium is stored can be increased or decreased according to the purpose, or it may be a replaceable vessel, with no particular restrictions.

In the cell culture method of the invention, culturing in which the number of cells in the cell culture vessel after culturing using the porous polymer film reaches $1.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more $5.0 \times 10^7$ or more, $1.0 \times 10^8$ or more, $2.0 \times 10^8$ or more, $5.0 \times 10^8$ or more, $1.0 \times 10^9$ or more, $2.0 \times 10^9$ or more, or $5.0 \times 10^9$ or more per milliliter of medium, assuming that all of the cells are evenly dispersed in the cell culture medium in the cell culture vessel, is mentioned.

It should be noted that as a method for measuring cell count during or after culture, various known methods may be used. For example, as the method for counting the number of cells in the cell culture vessel after culturing using the porous polymer film, assuming that the cells are evenly dispersed in the cell culture medium in the cell culture vessel, any publicly known method may be used. For example, a cell count method using CCK8 may be suitably used. Specifically, a Cell Counting Kit 8 (a solution reagent, commercially available from Dojindo Laboratories) (hereunder referred to as "CCK8") may be used to count the number of cells in ordinary culturing without using a porous polymer film, and the correlation coefficient between the absorbance and the actual cell count is determined. Subsequently, the cells are applied, the cultured porous polymer film may be transferred to CCK8-containing medium and stored in an incubator for 1 to 3 hours, and then the supernatant is extracted and its absorbance is measured at a wavelength of 480 nm, and the cell count is determined from the previously calculated correlation coefficient.

In addition, from another point of view, for example, "mass culturing of cells" may refer to culturing in which the number of cells in the cell culture vessel after culturing using the porous polymer film reaches $1.0 \times 10^5$ or more, $2.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more or $5.0 \times 10^7$ or more, $1.0 \times 10^8$ or more, $2.0 \times 10^8$ or more, or $5.0 \times 10^8$ or more, per square centimeter of porous polymer film. The number of cells contained per square centimeter of porous polymer film may be appropriately measured using a publicly known method, such as with a cell counter.

EXAMPLES

Hereinafter, the present invention will be described in more detail based on Examples. The present invention is not limited to the following Examples. Those skilled in the art can easily modify/modify the present invention based on the description of the present specification, and they are included in the technical scope of the present invention.

The porous polyimide films used in the following Examples were prepared by forming a polyamic acid solution composition including a polyamic acid solution obtained from 3,3',4,4'-biphenyltetracarboxylic dianhydride (s-BPDA) as a tetracarboxylic acid component and 4,4'-diaminodiphenyl ether (ODA) as a diamine component, and polyacrylamide as a coloring precursor, and performing heat treatment at 250°C or higher. The resulting porous polyimide film was a three-layer structure porous polyimide film having a surface layer A and a surface layer B, the surface layers having a plurality of pores, and a macrovoid layer sandwiched between the surface layers A and B; wherein the average pore diameter of the pore present on the surface layer A was 6 $\mu$m, the average pore diameter of the pore present on the surface layer B was 46 $\mu$m, and the film thickness was 25 $\mu$m, and the porosity was 73%.

[Example 1]

Cell culture method using a porous polyimide film with a cylindrical gas phase culture apparatus

Conditioned/suspended anti-human IL-8 antibody producing CHO-DP12 cells (ATCC CRL-12445) were suspension-cultured using a medium (BalanCD (Trademark) CHO GROWTH A) and culture was continued until viable cell count per mL was $9.9 \times 10^6$. Ten modules were placed in an oxygen permeable bag for shaking culture, and shaking culture was performed overnight in a $CO_2$ incubator.

On the next day, the module was taken out, and ten modules were placed in a cell culture unit of the cylindrical gas phase culture apparatus illustrated in FIG. 5 (medium discharge ports of each cell culture apparatus are parallel to each other). 350 mL of medium (KBM270 manufactured by Kohjin Bio Co., Ltd.) was pooled in a sump (corresponding to a medium storage tank 7 in FIG. 5), and the medium was circulated via a tube pump at a rate of 20 mL/min. When culture was terminated after 4 days, cells at a cell density of $2.5 \times 10^5$ cells/cm$^2$ with a total cell count of $5.0 \times 10^7$ were observed. The fact was demonstrated that a large amount of antibody-producing cells can be cultured with a compact and simple facility without using an oxygen supply apparatus.

[Example 2]

Cell culture method using a porous polyimide film with a cylindrical gas phase culture apparatus

Conditioned/suspended anti-human IL-8 antibody producing CHO-DP12 cells (ATCC CRL-12445) were suspension-cultured using a medium (BalanCD (Trademark) CHO GROWTH A) and culture was continued until viable cell count per mL was $2.4 \times 10^6$. 30 modules were sealed in an oxygen-permeable culture bag, the module having a mantle (casing) formed with a nylon mesh (30 #, mesh opening 547 $\mu$m) and having a fixed amount (20 cm$^2$ per module) of porous polyimide film aseptically added and sealed therein, and then 30 mL of the medium mentioned above was poured therein. After left in a $CO_2$ incubator overnight, on the next day, the module was taken out, and thirty modules were placed in a cell culture unit of the cylindrical gas phase culture apparatus illustrated in FIG. 5 (medium discharge ports of each of the cell culture apparatus are displaced in counterclockwise direction by 30 degree, see FIG. 4). 300 mL of medium (KBM270 manufactured by Kohjin Bio Co., Ltd.) was pooled in a sump (corresponding to a medium storage tank 7 in FIG. 5), and the medium was circulated via a tube pump at a rate of 20 mL/min.

When culture was terminated after 4 days, cells at a cell density of $7.1 \times 10^4$ cells/cm$^2$ with a total cell count of $5.8 \times 10^7$ were observed. The fact was demonstrated that a large amount of antibody-producing cells can be cultured with a compact and simple facility without using an oxygen supply apparatus.

[Example 3]

Cell culture method using a porous polyimide film with a cell culture apparatus provided with a medium droplet supplying means (hereinafter referred to as "a mist and shower-type reactor")

Conditioned/suspended anti-human IL-8 antibody producing CHO-DP12 cells (ATCC CRL-12445) were suspension-cultured using a medium (BalanCD (Trademark) CHO GROWTH A) and culture was continued until viable cell count per mL was $3.9 \times 106$. After the suspension culture medium (12 mL each) was poured onto one dish (diameter, 10 cm), 12 modules were added to the dish, the modules having a mantle (casing) formed with a nylon mesh (30 #, mesh opening 547 $\mu$m) and having a fixed amount (20 cm$^2$ per module) of porous polyimide film aseptically added and sealed therein. The module was wetted with the cell suspension and then left overnight in a $CO_2$ incubator.

On the next day, the module was taken out and 12 modules were placed on the stage part of the mist and shower type reactor (see, FIG. 9(A)), 200 mL of medium (IMDM containing 2% FBS) was pooled in a medium storage tank, and the medium was circulated via a tube pump at a rate of 60 mL/min. When culture was terminated after 2 days, cells at a cell density of $3.4 \times 10^4$ cells/cm$^2$ with a total cell count of $8.2 \times 10^6$ were observed.

[Example 4]

Cell culture method with mist and shower type reactor using porous polyimide film

Conditioned/suspended anti-human IL-8 antibody producing CHO-DP12 cells (ATCC CRL-12445) were suspension-cultured using a medium (BalanCD (Trademark) CHO GROWTH A) and culture was continued until viable cell count per mL was $9.9 \times 10^6$. Ten modules were placed in an oxygen permeable bag for shaking culture, and shaking culture was performed overnight in a $CO_2$ incubator.

On the next day, the module was taken out from the shaking bag and subjected to cell culture with a mist and shower type reactor under the same conditions as in Example 4. 200 mL of medium (KBM270 manufactured by Kohjin Bio Co., Ltd.) was pooled in a medium storage tank, and the medium was circulated via a tube pump at a rate of 60 mL/min. When culture was terminated after 4 days, cells at a cell density of $8.8 \times 10^4$ cells/cm$^2$ with a total cell count of $1.8 \times 10^7$ were observed.

[Example 5]

Preparation of a cylindrical gas phase culture apparatus (hereinafter referred to as "a gas phase cylinder type bioreactor") with a modularized porous polymer film having a stainless steel casing (hereinafter referred to as "a metal module"), and cell culture using the apparatus

In order to fully utilize the heat resistance of the porous polyimide film and complete the sterilization operation by a simple bulk dry heat sterilization, a metal module composed of a stainless steel mesh casing, a liner, and a porous polyimide film was prepared (see, FIG. 10). Specifically, a laminate of a 1 cm $\times$ 1 cm porous polyimide film and a porous polyimide film laminated with a stainless steel mesh (referred to as "liner", not illustrated) having the same area (3 porous polyimide films, 1 liner, 4 porous polyimide films, 1 liner, 3 porous polyimide films, stacked in this order) were sealed in a stainless steel mesh casing to prepare a metal module (FIG. 10). The operation was performed in a non-sterilized fashion in an open space.

In the glass heat-resistant gas phase cylinder type reactor for operating the metal module, a metal module is placed in the glass chamber, and the medium can be supplied into the vapor cylinder by dropping. Since a gas phase cylinder type bioreactor equipped with a metal module is exclusively made of a heat resistant material, sterilization can be performed only using simple dry heat sterilization. After 30 metal modules were placed in a heat-resistant vapor cylinder, the apparatus aseptically assembled was wrapped with aluminum foil, dry heat sterilized at 190°C for 80 minutes, and allowed to cool to complete sterilization.

Using the prepared gas phase cylinder type bioreactor, experiment for culturing human skin fibroblasts was started.

As described above, the entire gas phase cylinder type reactor was assembled aseptically and the whole apparatus was placed in a $CO_2$ incubator. Human skin fibroblasts cultured on a dish were detached by trypsin treatment to prepare a cell suspension (70 mL, each) for the respective reaction embodiment depicted in FIG. 8. The cell density per 1 mL at this point was $1.4 \times 10^5$. The cell proliferation behavior after adsorption is described in Table 2.

[Table 2]

| Medium Addition Method | Liquid Supply (1) Drop-type (FIG. 8(A)) | Liquid Supply (2) Mesh-type (FIG. 8(A)) | Liquid Supply (3) Shower-type (FIG. 8(C)) |
|---|---|---|---|
| Residual Cell Density in Liquid Cells/mL (*1) | Below Detection Limit | $1.4 \times 10^4$ | Below Detection Limit |
| Cell Adsorption (*2) | ~100% | 90% | ~100% |
| Expected Initial Maturity (Compared with Maximum Value) (*3) | 15% | 14% | 15% |
| Maturity on Day 5 (*4) | 12.8% (Upper) 10.2% (Lower) | 26.7% | 19.3% (Upper) 20.8% (Lower) |

* 1: The residual cell density in the liquid indicates the number of cells (density) remaining in the cell suspension after absorbing the cells to the porous polyimide film.
* 2: Cell adsorption ratio indicates how much cells in the cell suspension used for seeding have been adsorbed on the porous polyimide film.
* 3: Expected initial maturity is expressed as the number of adsorbed cells in actual cells as %, assuming that the maximum population of cells in the porous polyimide film is 100%.
* 4: The same as *3 (calculated on Day 5 of culture). Upper; the value of the top module, Lower; the value of the top module, respectively.

As depicted in FIG. 8(D), a drop type droplet was attained by introducing a rolled-up stainless steel mesh (product number E 9103, 20#, manufactured by Kyuho Corporation, Japan) (corresponding to a mesh bundle in FIG. 8(D)) through a medium supplying port provided in a lid body. In addition to the drop type method, droplet of a mesh type was attained by providing a planar stainless steel mesh (product number: E9103, 20 #, manufactured by Kyuho Corporation, Japan) directly under the mesh bundle to cover the module. Droplet of a shower type was attained by using a nozzle of product number 1/8 MVVP 6503 PP-IN manufactured by H. IKEUCHI Co., Ltd.

Thereafter, by continuously supplying a medium (using KBM Fibro Assist manufactured by Kohjin Bio Co., Ltd.) using a pump, circulation of the medium was started and continuous culture was performed. Culture was continued while exchanging medium once every 3 days. Regarding the evaluation of the cell count, 1 to 2 modules were taken out of 30 modules, and cell count of human skin dermal fibroblasts growing in those modules was measured using the color reaction of Cell Counting Kit8; a solution reagent manufactured by Dojindo Laboratories (hereunder referred to as "CCK8"). As is apparent from this experimental result, in this experiment, it was found that how to pour the medium determines the cell count in the module in each of the subsequent system. Interestingly, it was also observed that the effect of leveling of the liquid penetration by a mesh was very great, resulting in steady proliferation of cells. On the other hand, in the drop addition method, it was thought that the medium failed to spread throughout the inside of the reactor, and the cell growth region was limited due to the drift, which caused the decreased cell count. It is thought that the method of pouring the culture medium in a shower-like manner also contributes liquid leveling effect to some degree.

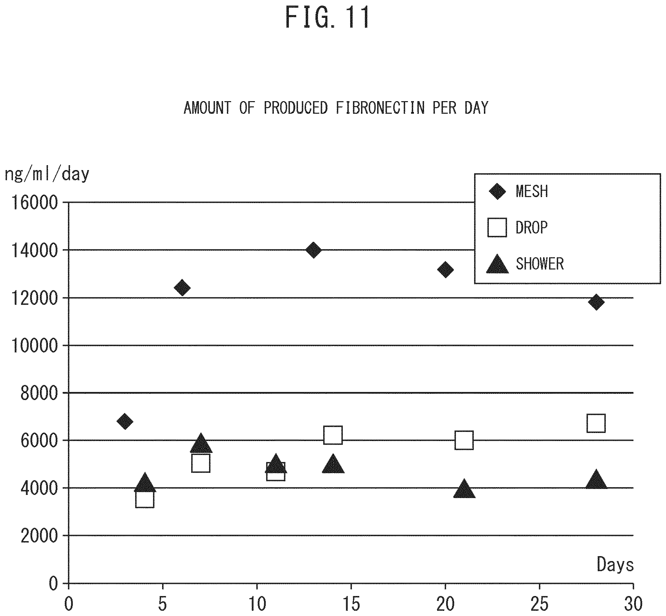

Subsequently, in order to verify efficiency of a bioreactor in the present culture method, we proceeded with evaluation of substance productivity. ELISA-kit for measuring human fibronectin manufactured by Takara Bio Inc. was used to measure the amount of the produced fibronectin. The measurement results are depicted in FIG. 11.

As is obvious from FIG. 11, a mesh culture method overwhelmingly predominates also in this substance production evaluation, and has been steadily increasing fibronectin productivity. Stable operation for 1 month could be attained. On the other hand, also in the shower type and drop type apparatuses, stable substances were attained, but improvement in productivity could not be observed. It was possible to demonstrate that gas phase exposed type culture enabled stable culture of human primary cells and high efficient production of valuable substances, using a very simple apparatus.

REFERENCE SIGNS LIST

| | |
|---|---|
| 1, 1', 1' | Cell culture apparatus |
| 2, 2a to 2e, 2' | Cell culture unit |
| 21, 21a to 21e | Side section |
| 22, 22a, to 22e | Bottom section |
| 23, 23a to 23e | Medium discharge port |
| 24 | Fastener inserting port |
| 25 | Upper section |
| 26 | Medium supplying port |
| 27 | Lid section |
| 271 | Medium discharge port |
| 3 | Medium supplying means |
| 30 | Medium storage unit |
| 31 | Side section |
| 32 | Bottom section |
| 33 | Medium dropping nozzle |
| 330 | Nozzle port |
| 331 | Droplet |
| 34 | Fastener inserting port |
| 35 | Medium dropping port |
| 36 | Overflow pipe |
| 37 | Leg section |
| 3', 3'a, 3'b | Medium droplet supplying means |
| 30' | droplet mesh |
| 31' | Mesh bundle |
| 4, 4' | Medium collecting means |
| 4'a | Cell culture unit/medium collecting means |
| 41, 41', 41'a | Medium discharging section |
| 5, 5', 5'a | Outer cylinder |
| 51 | Stopper |
| 52 | Outer cylinder lid section |
| 52a | Lid section medium supplying port |
| 61 | Fastener |
| 7 | Medium storage tank |
| 71 | Medium |
| 72 | Medium discharging line |
| 73 | Medium supplying line |
| 8 | Pump |
| 9 | Porous polymer film |
| 90 | Modularized porous polymer film |
| 900 | Casing |

**Claims**

1.  A cell culture apparatus comprising:

    a porous polymer film;
    a cell culture section containing the porous polymer film;
    a medium supplying means disposed at a upper portion of the cell culture section; and
    a medium collecting means disposed at a lower portion of the cell culture section,

    wherein the porous polymer films are a three-layer structure porous polymer film having a surface layer A and a surface layer B, the surface layers having a plurality of pores, and a macrovoid layer sandwiched between the surface layers A and B;
    wherein an average pore diameter of the pores present in the surface layer A is smaller than an average pore diameter of the pores present in the surface layer B;
    wherein the macrovoid layer has a partition wall bonded to the surface layers A and B, and a plurality of macrovoids surrounded by such a partition wall and the surface layers A and B;
    wherein the pores in the surface layers A and B communicate with the macrovoid; and
    wherein the cell culture section comprises a bottom portion having one or more medium outlets, and a side portion disposed approximately perpendicular to the bottom portion.

2.  The cell culture apparatus according to claim 1, wherein two or more cell culture sections are stacked.

3.  The cell culture apparatus according to claim 1 or 2, wherein the side portion has one or more additional medium inlets.

4.  The cell culture apparatus according to any one of claims 1 to 3, the apparatus **characterized by** further comprising:

    a medium discharging line communicating with the medium collecting means at one end;
    a medium storage tank communicating with the other end of the medium discharging line; and
    a medium supplying line communicating with the medium storage tank at one end;

    wherein the other end of the medium supplying line communicates with the medium supplying means, and the medium circulates.

5.  The cell culture apparatus according to claim 4, the apparatus further comprising a pump for pumping up the medium in the medium storage tank into the medium supplying means.

6.  The cell culture apparatus according to any one of claims 1 to 5, wherein the medium collecting means is funnel-shaped.

7.  The cell culture apparatus according to any one of claims 1 to 6, wherein the medium supplying means has a medium storage section, and two or more medium dropping nozzles provided at the bottom portion of the medium storage section.

8.  The cell culture apparatus according to any one of claims 1 to 6, wherein the medium supplying means is a medium droplet supplying means.

9.  The cell culture apparatus according to any one of claims 1 to 8, the apparatus further comprising:
    an outer cylinder for housing the cell culture section;
    wherein the medium supplying means is disposed in the outer cylinder and above the cell culture section;
    wherein the culture medium collecting means is disposed in the outer cylinder and under the cell culture section.

10. The cell culture apparatus according to any one of claims 1 to 9, wherein the porous polymer film is contained in the cell culture section, with the porous polymer film:

    i) being folded up;
    ii) being wound into a roll-like shape;
    iii) sheets or pieces thereof being concatenated with a thread-like structure;
    iv) being tied together into a rope-like shape; and/or

v) two or more thereof being stacked.

11. The cell culture apparatus according to any one of claims 1 to 9, wherein the porous polymer film is a modularized porous polymer film having a casing;
wherein the modularized porous polymer film is contained within the casing with:

(i) the two or more independent porous polymer films being aggregated;
(ii) the porous polymer film being folded up;
(iii) the porous polymer film being wound into a roll-like shape; and/or
(iv) the porous polymer film being tied together into a rope-like shape;

wherein the modularized porous polymer film is contained in the cell culture section.

12. The cell culture apparatus according to any one of claims 1 to 11, wherein the porous polymer film has a plurality of pores having an average pore diameter of 0.01 to 100 $\mu$m.

13. The cell culture apparatus according to any one of claims 1 to 12, wherein an average pore diameter of the surface layer A is 0.01 to 50 $\mu$m.

14. The cell culture apparatus according to any one of claims 1 to 13, wherein an average pore diameter of the surface layer B is 20 to 100 $\mu$m.

15. The cell culture apparatus according to any one of claims 1 to 14, wherein a total film thickness of the porous polymer film is 5 to 500 $\mu$m.

16. The cell culture apparatus according to any one of claims 1 to 15, wherein the porous polymer film is a porous polyimide film.

17. The cell culture apparatus according to claim 16, wherein the porous polyimide film is a porous polyimide film comprising a polyimide derived from tetracarboxylic dianhydride and diamine.

18. The cell culture apparatus according to claim 16 or 17, wherein the porous polyimide film is a colored porous polyimide film that is obtained by molding a polyamic acid solution composition comprising a polyamic acid solution derived from tetracarboxylic dianhydride and diamine, and a coloring precursor, and subsequently heat-treating the resultant composition at 250°C or higher.

19. The cell culture apparatus according to any one of claims 1 to 15, wherein the porous polymer film is a porous polyethersulfone film.

20. A method for culturing a cell which uses the cell culture apparatus according to any one of claims 1 to 19.

21. A cell culture apparatus comprising:

a porous polymer film;
a cell culture section containing the porous polymer film;
a medium supplying means disposed at a upper portion of the cell culture section; and
a medium collecting means disposed at a lower portion of the cell culture section,

wherein the porous polymer films are a three-layer structure porous polymer film having a surface layer A and a surface layer B, the surface layers having a plurality of pores, and a macrovoid layer sandwiched between the surface layers A and B;
wherein an average pore diameter of the pores present in the surface layer A is smaller than an average pore diameter of the pores present in the surface layer B;
wherein the macrovoid layer has a partition wall bonded to the surface layers A and B, and a plurality of macrovoids surrounded by such a partition wall and the surface layers A and B;
wherein the pores in the surface layers A and B communicate with the macrovoid; and
wherein the medium collecting means is a part of an outer cylinder for housing the cell culture section.

**22.** The cell culture apparatus according to claim 21, wherein the medium supplying means is a medium droplet supplying means.

**23.** The cell culture apparatus according to claim 21 or 22, wherein the porous polymer film is a modularized porous polymer film having a casing;
wherein the modularized porous polymer film is contained within the casing with:

(i) the two or more independent porous polymer films being aggregated;
(ii) the porous polymer film being folded up;
(iii) the porous polymer film being wound into a roll-like shape; and/or
(iv) the porous polymer film being tied together into a rope-like shape;

wherein the modularized porous polymer film is contained in the cell culture section.

# FIG. 1

FIG. 2

# FIG. 3

(A)

(C)

(B)

EP 3 489 347 A1

# FIG. 4

# FIG. 5

FIG. 6

MESH SURFACE

LARGE PORE SURFACE

APPARENT MEMBER VOLUME

MEMBER VOLUME CONTAINING CELL SURVIVAL ZONE

FIG. 7

FIG. 8

(A)　　　　　　(B)　　　　　　(C)　　　　　　(D)

3'a

331

3'a

331

30'

3'b

331

52

52a

31'

EP 3 489 347 A1

FIG. 9

# FIG. 10

# FIG. 11

AMOUNT OF PRODUCED FIBRONECTIN PER DAY

## INTERNATIONAL SEARCH REPORT

| International application No. |
|---|
| PCT/JP2017/026945 |

**A. CLASSIFICATION OF SUBJECT MATTER**
*C12M3/00*(2006.01)i, *C12N5/071*(2010.01)i

According to International Patent Classification (IPC) or to both national classification and IPC

**B. FIELDS SEARCHED**

Minimum documentation searched (classification system followed by classification symbols)
C12M3/00, C12N5/071

Documentation searched other than minimum documentation to the extent that such documents are included in the fields searched
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| P,X | WO 2016/121775 A1 (Ube Industries, Ltd.), 04 August 2016 (04.08.2016), claims 1 to 37; paragraphs [0065], [0068] & CA 2974278 A | 1-23 |
| X | WO 2015/012415 A1 (Ube Industries, Ltd.), 29 January 2015 (29.01.2015), claims 1 to 23; paragraphs [0057], [0093] to [0095], [0100] to [0101] & US 2016/0168560 A particularly, claims 1 to 23; paragraphs [0098], [0137] to [0139], [0144] to [0145] & EP 3026108 A1      & CN 105452440 A & KR 10-2016-0034303 A | 1-6,9-21,23 |
| A | | 7-8,22 |

☒ Further documents are listed in the continuation of Box C.  ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered    to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 06 October 2017 (06.10.17) | 17 October 2017 (17.10.17) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

| International application No. |
|---|
| PCT/JP2017/026945 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2006/136953 A2  (CAPSANT NEUROTECHNOLOGIES LTD.), 28 December 2006 (28.12.2006), claim 6 & JP 2008-543303 A    & US 2009/0042288 A1 & EP 1896572 A2    & CN 101268184 A | 1-23 |
| A | JP 2012-175964 A  (IHI Corp.), 13 September 2012 (13.09.2012), claims 1 to 11; fig. 1 to 6 (Family: none) | 1-23 |
| A | JP 2013-021976 A  (IHI Corp.), 04 February 2013 (04.02.2013), claims 1 to 10; fig. 1 to 3 (Family: none) | 1-23 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003054174 A
- WO 2010038873 A
- JP 2011219585 A
- JP 2011219586 A
- WO 2015012415 A
- WO 2009123349 A
- JP 2009057041 W